# EUROPEAN PATENT APPLICATION

(11) **EP 4 275 641 A1**
(43) Date of publication of application: **15.11.2023**
(21) Application number: 22173051.8
(22) Date of filing: 12.05.2022
(51) Int. Cl.: A61B 34/10, A61B 34/20

(54) **TECHNIQUE FOR VISUALIZING A PLANNED IMPLANT**

(71) Applicant: Stryker European Operations Limited, Carrigtwohill, Co. Cork T45 HX08 (IE)
(72) Inventor: WEEDE, Oliver, 79110 Freiburg (DE); KASTROP, Manuel, 10437 Berlin (DE); MANUEL MARTINS, Maria, 68420 Eguisheim (FR)
(74) Representative: Röthinger, Rainer

(57) **Abstract**

The present disclosure relates to a method for visualizing a planned implant. The method comprises obtaining image data representative of an anatomical element, obtaining implant data indicative of geometrical properties of an implant to be implanted into the anatomical element, and obtaining planning data indicative of a planned pose of the implant. Based on the obtained data, a first part of the implant that lies inside the anatomical element, and a second part of the implant that does not lie inside the at least one anatomical element are determined. Finally, display of a visualization indicating the first part distinguishably from the second part is triggered. The disclosure also relates to a system and a computer program.

## Description

### Technical Field

The present disclosure generally relates to a method for visualizing a planned implant. The present disclosure also provides for a system and a computer program.

### Background

In certain surgical scenarios, a connection portion of a first implant should be arranged within a first anatomical element such as a bone of a patient. Other portions of the first implant, for example an implant interface to be connected to a second anatomical element, a second implant or an implant connector, may need to be arranged outside the first anatomical element. The arrangement of the first implant may also need to comply with restrictions posed on an incision point and an insertion direction of the first implant along which the first implant is to be inserted into the patient's body.

In computer-assisted surgery (CAS), a surgeon may be provided with a view that visualizes an implant in a planned pose relative to patient image data. The patient image data may be medical image data obtained from a computer tomography (CT) device or a magnetic resonance (MR) imaging device, for example. Using currently available views that visualize the implant in the planned pose relative to the patient image data, it may be difficult for the surgeon to judge whether the planned pose is acceptable or not. For example, it may be hard to identify whether the connecting portion of the implant in the planned pose extends through critical zones comprising artilleries, nerve tissue and the like, or whether the complete connecting portion of the implant is arranged inside the first anatomical element as intended. It may also be difficult to decide, based on currently known visualizations, whether the planned pose or a geometric property of the implant (e.g., a length, diameter or shape) needs to be adjusted, and, if so, how. Still further, it may be hard for a surgeon to determine whether an incision point, through which the implant needs to be inserted into the patient's body to be placed in the planned pose, is acceptable or not. Still further, it may be hard for a surgeon to predict whether the implant will collide with an obstacle like a bony structure when it is being inserted into the patient's body later on.

### Summary

There is a need for a technique that solves one or more of the aforementioned or other problems.

According to a first aspect, a method for visualizing a planned implant is provided. The method is performed by a processing system and comprises obtaining image data representative of a portion of a patient's body, the portion comprising at least one anatomical element. The method comprises obtaining implant data indicative of one or more geometrical properties of an implant to be implanted into the at least one anatomical element, and obtaining planning data indicative of a planned pose of the implant relative to the at least one anatomical element (e.g., represented by the image data). The method further comprises determining, based on the image data, the implant data and the planning data, at least one first part of the implant that lies inside the at least one anatomical element (e.g., represented by the image data) and at least one second part of the implant does not lie inside the at least one anatomical element (e.g., represented by the image data). The method comprises triggering display of a visualization indicating the at least one first part distinguishably from the at least one second part.

The image data may comprise medial image data. The image data may be generated by a medical imaging device such as a CT device or a MR imaging device. The image data may comprise CT image data. Alternatively, or in addition, the image data may comprise MR image data. The image data may comprise or consist of pre-operative image data or intra-operative image data. The image data may be representative of both (i) the portion of the patient's body and (ii) all remaining portions of the patient's body.

According to the present disclosure, a 'pose' may comprise at least one of a position and an orientation. For example, the planned pose of the implant may comprise a position and orientation of the implant relative to the at least one anatomical element (e.g., as represented by the image data).

The method may comprise a step of determining the visualization based on the determined at least one first part and the determined at least one second part. The visualization may guide a surgeon on how to adapt the planned pose by distinguishably visualizing the at least one first part from the at least one second part. The visualization may provide the surgeon with information on parts of the implant that lie within the at least one anatomical element, and parts of the implant that lie outside the at least one anatomical element such that the surgeon may not need to cognitively determine these parts by himself. The visualization may guide the surgeon on how to adapt the pose of the implant such that the at least one second part is minimized in size, such that a portion of the at least one second part is changed into a portion of the at least one first part or such that the at least one first part is maximized in size, for example.

The method may be a computer-implemented method. The method may be performed by at least one processor instead of the processing system. Alternatively, the processing system may comprise at least one processor configured to carry out the method. The method does not comprise a surgical step.

The at least one second part may lie inside at least one zone separate from the at least one anatomical element.

The at least one zone may comprise a zone distant from one or more of the at least one anatomical element. Alternatively, or in addition, the at least one zone may , comprise a zone adjacent to one or more of the at least one anatomical element.

The visualization may indicate a first segment of the at least one second part that lies inside a first zone of the at least one zone distinguishably from a second segment of the at least one second part that lies inside a second zone of the at least one zone. The first zone may be different from the second zone.

The visualization may indicate a third segment of the at least one second part that is comprised in a first predefined section of the implant distinguishably from a fourth segment of the at least one second part that is comprised in a second predefined section of the implant, wherein the first predefined section differs from the second predefined section.

The visualization may be an augmented view.

The method may comprise obtaining a first registration between (i) the image data and (ii) one or more of the at least one anatomical element. The method may further comprise obtaining a second registration between (i) an augmented reality device comprising a display and (ii) one or more of the at least one anatomical element. The visualization may be determined based on the first registration and the second registration. An orientation or viewing direction of the visualization may be determined based on the first registration and the second registration. The visualization may be triggered to be displayed on the display of the augmented reality device.

The method may comprise obtaining a surface scan of (e.g., at least the portion of) the patient's body. The method may comprise determining, based on the surface scan and the planning data, an incision point on the surface of the patient's body at which an incision is to be made for inserting the implant into the patient's body. The visualization may further indicate the incision point.

The incision point may be determined as an intersection between (i) a surface of the patient's body indicated by the surface scan and (ii) a trajectory having a predefined pose relative to the implant.

The surface scan may be acquired by a sensor comprised in the augmented reality device.

The method may further comprise, after having triggered display of the visualization, obtaining a user input indicative of an updated planned pose of the implant relative to the at least one anatomical element (e.g., represented by the image data). The method may comprise updating the visualization based on the updated planned pose.

The method may further comprise identifying the at least one anatomical element in the image data. The method may comprise defining the at least one zone based on the identified at least one anatomical element.

The planned pose may be defined relative to the image data. The planning data may be indicative of a planned pose of the implant relative to the at least one anatomical element as represented by the image data.

The method may further comprise identifying the at least one anatomical element in the image data, and determining the planned pose based on the identified at least one anatomical element and one or more predefined spatial constraints between the implant and the identified at least one anatomical element.

According to a second aspect, a method is provided. The method comprises obtaining planning data indicative of a planned pose of an implant relative to at least one anatomical element comprised in a portion of a patient's body. The method further comprises obtaining a surface scan of at least the portion of the patient's body. The method may comprise determining, based on the surface scan and the planning data, an incision point on the surface of the patient's body at which an incision is to be made for inserting the implant into the patient's body. The method may comprise triggering display of a visualization indicating the incision point.

The surface scan may be separate from (e.g., not included in) the image data. The surface scan may be non-medical image data. The surface scan may represent only the surface of at least the portion of the patient's body. The surface scan may consist of an image representing only the surface of at least the portion of the patient's body. The surface scan may be obtained from a depth sensor such as a time-of-flight sensor or a stereo camera. The surface scan may be acquired by a sensor comprised in an augmented reality device.

The method may comprise a step of registering the surface scan to the image data or vice versa, for example based on a third registration (e.g., between (i) a coordinate system of the image data and (ii) a coordinate system of the surface scan or the sensor acquiring the surface scan). The third registration may be determined based on a tracked pose of the at least one anatomical element, a (e.g., the) first registration between the image data and the at least one anatomical element and a tracked pose of the sensor or scanner that acquires the surface scan. Other variants for determining the third registration are also possible.

The visualization may be an augmented view. The visualization may be triggered to be displayed on a display of an (e.g., the) augmented reality device. The incision point may be determined as an intersection between (i) a surface of the patient's body indicated or represented by the surface scan and (ii) a trajectory having a predefined pose relative to the implant. The trajectory may coincide with a longitudinal axis of the implant. The implant may be a pedicle screw.

The method of the second aspect may comprise one or more additional steps of the method of the first aspect. The image data may correspond to the image data described for the first aspect. The planning data may correspond to the planning data described for the first aspect. The implant may correspond to the implant described for the first aspect. The at least one anatomical element may correspond to the at least one anatomical element described for the first aspect. The surface scan may correspond to the surface scan described for the first aspect. The augmented reality device may correspond to the augmented reality device described for the first aspect. The visualization may comprise additional indications and content, as described with respect to the visualization according to the first aspect.

According to a third aspect, a system is provided. The system comprises at least one processor configured to perform a method for visualizing a planned implant. The at least one processor is configured to obtain image data representative of a portion of a patient's body, the portion comprising at least one anatomical element, obtain implant data indicative of one or more geometrical properties of an implant to be implanted into the at least one anatomical element, obtain planning data indicative of a planned pose of the implant relative to the at least one anatomical element (e.g., as represented by the image data), determine, based on the image data, the implant data and the planning data, at least one first part of the implant that lies inside the at least one anatomical element (e.g., as represented by the image data) and at least one second part of the implant does not lie inside the at least one anatomical element (e.g., as represented by the image data), and trigger display of a visualization indicating the at least one first part distinguishably from the at least one second part.

The system may be referred to as a processing system or as a CAS system.

The at least one processor may be configured to perform the method according to the first aspect. Alternatively, or in addition, the at least one processor may be configured to perform the method according to the second aspect.
The system may further comprise an augmented reality device comprising a display. The at least one processor may be configured to trigger display of the visualization on the display of the augmented reality device. The augmented reality device may correspond to the augmented reality device described for the first aspect.

The system may further comprise a tracking system configured to track the patient's body and the augmented reality device to determine a relative pose between the patient's body and the augmented reality device. The tracking system may correspond to the surgical tracking system described for the first aspect.

According to a fourth aspect, a computer program is provided. The computer program comprises instructions which, when executed on at least one processor, cause the at least one processor to perform the method according to at least one of the first aspect and the second aspect. The computer program may be stored on one or more (e.g., non-transitory) computer-readably media, or may be carried by a carrier such as a data stream.

### Brief Description of the Drawings

Further details, advantages and aspects of the present disclosure will become apparent from the following embodiments taken in conjunction with the drawings, wherein:
- Fig. 1: shows a system in accordance with the present disclosure;
- Fig. 2: shows a method in accordance with the present disclosure;
- Fig. 3a-3f: show visualizations in accordance with the present disclosure.

### Detailed Description

In the following description, exemplary embodiments of a system and a method will be explained with reference to the drawings. The same reference numerals will be used to denote the same or similar structural features.

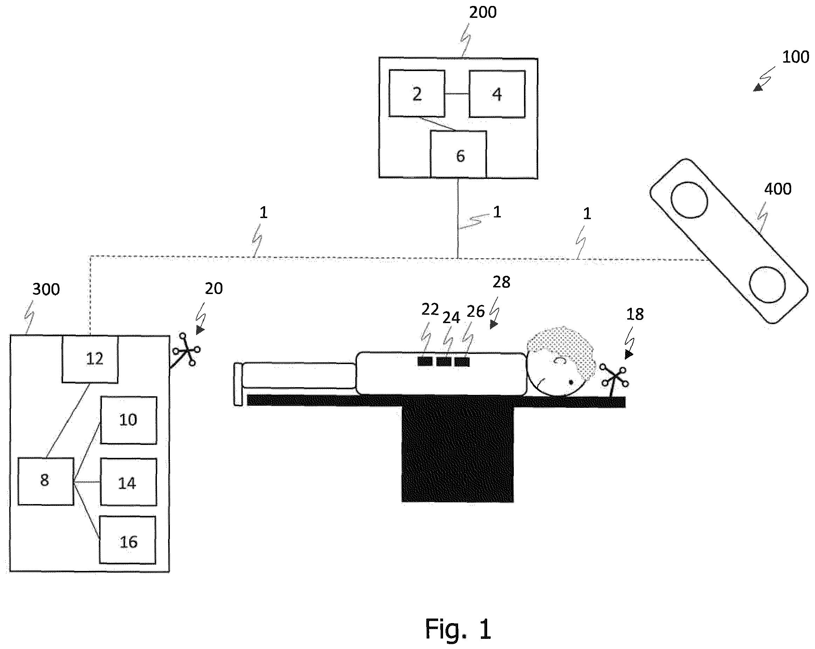

Fig. 1 shows a system 100 in accordance with the present disclosure. The system 100 comprises a computing system 200, an augmented reality device (ARD) 300 and a surgical tracking system (STS) 400, connected to one another via wired or wireless connections 1. The system 100 may be referred to as a processing system or as a CAS system.

The computing system 200 comprises a processor 2, coupled to a memory 4 and an interface 6. The memory comprises instructions that, when performed by the processor 2, cause the processor 2 to carry out the method(s) described herein. The processor 2 may obtain data via the interface 6 and may transmit data and trigger commands via the interface 6. In one variant, the computing system 200 comprising the processor 2 may be referred to as a processing system.

The ARD 300 comprises a processor 8, a memory 10 and an interface 12. The processor 8 may receive and transmit data via the interface 12. The ARD 300 further comprises a display 14 and at least one sensor 16. The display 14 is configured to provide a user of the ARD with a visualization. The at least one sensor 16 may comprise one or more of a camera, a stereo camera, a time-of-flight camera, a Lidar sensor, and a depth sensor. The ARD 300 may be a head-mountable device (HMD).

In one variant, the ARD 300 comprising the processor 8 may be referred to as a processing system.

In one implementation, the processor 2 is configured to carry out the computer-implemented method(s) described herein. Instead of the processor 2, the processor 8 of the ARD 300 may be configured to carry out the computer-implemented method(s) described herein. In another variant, some of the steps of the method(s) described herein may be performed by the processor 2, and other steps of the method(s) described herein may be performed by the processor 8.

The STS 400 in the illustrated example is an optical tracking system comprising a stereo camera configured to track one or more surgical trackers. Each surgical tracker may comprise a plurality of optical tracking markers detectable in images acquired by the stereo camera. The STS 400 is configured to track a patient tracker 18 fixedly attached relative to the patient's body 28, and an ARD tracker 20 fixedly attached relative to the ARD 300, to determine a pose of each tracker in a tracking coordinate system. Put differently, the STS 400 may track (e.g., the anatomical elements 22, 24, 26 of) the patient's body 28 via the tracker 18, and may track the ARD 300 via the ARD tracker 20. The STS 400 may also track a surgical instrument such as a registration probe, via an instrument tracker attached to the surgical instrument (not shown).

Instead of tracking the trackers 18, 20, the STS 400 may directly track (e.g., the anatomical elements 22, 24, 26 of) the patient's body 28. The STS 400 may directly track the ARD 300. To this end, the STS 400 may employ one or more of image analysis, feature detection, object recognition and machine vision.

Instead of tracking the ARD 300 or the tracker 20, the pose of the ARD 300 may be determined relative to the STS 400 based on sensor data acquired with the sensor(s) 16. The sensor(s) 16 may be used to track the patient tracker 18 or to directly track (e.g., the anatomical elements 22, 24, 26 of) the patient's body 28.

The STS 400 is not limited to an optical tracking system. The STS 400 may be an electromagnetic tracking system, for example.

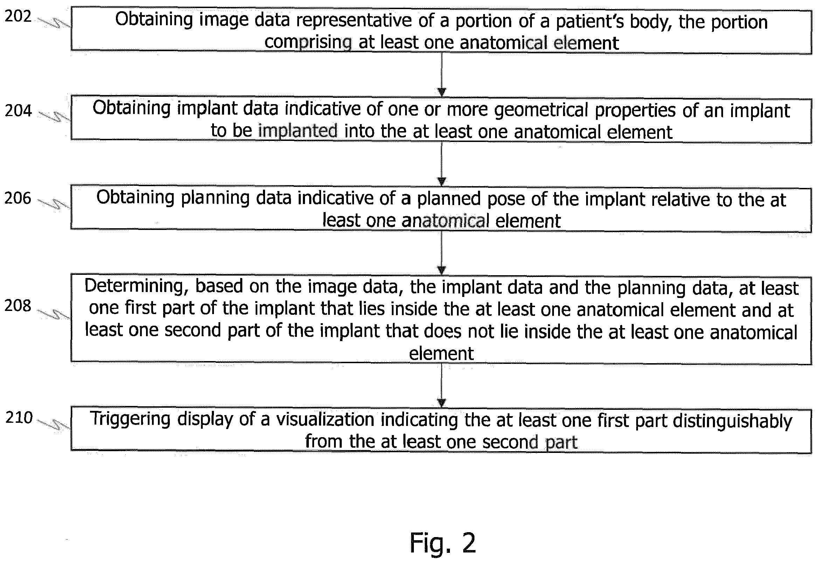

Fig. 2 shows a method in accordance with the present disclosure. As noted above, at least one step of the method may be performed by the processor 2, and at least one step of the method may be performed by the processor 8. The processors 2, 8 may transmit data to one another. For example, the processor 2 may obtain the image data from a medical imaging device and transmit the image data to the processor 8. In one exemplary implementation, all steps of the method are performed by exactly one of the processors 2, 8. In a preferred implementation, all steps of the method are performed by the processor 2. This may reduce computational requirements and energy consumption of the processor 8.

In a step 202, image data is obtained. The image data is representative of a portion of the body 28 of the patient, the portion comprising at least one anatomical element, for example the anatomical element 22.

The image data may also be referred to as patient image data. The image data may comprise medial image data. The image data may be generated by a medical imaging device such as a CT device or a MR imaging device. The image data may comprise CT image data. Alternatively, or in addition, the image data may comprise MR image data. The image data may be pre-operative image data or intra-operative image data.

The at least one anatomical element 22 may correspond to a region of the patient's body 28 that has predefined biological properties, e.g., comprises or consists of a certain type of (e.g., healthy) body cells. The at least one anatomical element 22 may exclude tumor tissue. The at least one anatomical element 22 may correspond to a (e.g., the) region of the patient's body 28 that has predefined physical properties, e.g., a predefined (e.g., maximum or minimum) size, a predefined shape, a predefined (e.g., maximum or minimum) volume, a water concentration exceeding a predefined threshold, a water concentration below a predefined threshold or an x-ray absorption rate meeting a predefined criterion (e.g., exceeding a predefined Hounsfield threshold). The at least one anatomical element 22 may comprise or be an organ or a bone. The at least one anatomical element 22 may comprise or consist of a bone (e.g., as represented by or identified in the image data) and a safety zone (e.g., with a predefined thickness) that surrounds the bone. The safety zone may compensate for inaccuracies of at least one of (i) the image data and (ii) the identification of the bone based on the image data. The bone may be a vertebra.

In step 204, implant data is obtained. The implant data is indicative of one or more geometrical properties of an implant to be implanted into the at least one anatomical element 22.

The implant may comprise a portion to be inserted into the at least one anatomical element 22. The implant may extend along a longitudinal axis. The implant or the portion to be inserted may comprise or consist of a bone screw, such as a pedicle screw. The one or more geometrical properties of the implant may comprise at least one parameter selected from a size, a length, a diameter, a radius and a shape of the implant. The one or more geometrical properties of the implant may define an outer surface or contour of the implant.

In step 206, planning data is obtained. The planning data is indicative of a planned pose of the implant relative to the at least one anatomical element 22 (e.g., as represented by the image data).

The planned pose may be defined relative to the image data or may be defined in the image data. The implant may be planned in an image comprised in or generated based on the image data. The predefined pose may be defined in an image coordinate system of an image comprised in or generated based on the image data.

The planned pose may be transformed from a planning coordinate system into the tracking coordinate system or into the image coordinate system using one or more predetermined coordinate transformations, also referred to as registrations. In another variant, the planned pose may be defined relative to the at least one anatomical element 22 of the patient's body 28 in the tracking coordinate system of the STS 400. A spatial relationship between the planned pose and the at least one anatomical element as represented by the image data may then be determined based on a first registration as discussed further below.

The method may further comprise identifying the at least one anatomical element 22 in the image data. The at least one anatomical element 22 may be identified based on image segmentation. The at least one anatomical element 22 may be identified by detecting, in (e.g., one or more images of) the image data, a region that exhibits a predefined property. The predefined property may be at least one of a biological and a physical property as described above. The at least one anatomical element may be identified using an anatomical atlas. The at least one anatomical element may be identified based on the image data using an artificial neural network trained with image data sets of corresponding anatomical elements that have been identified (e.g., marked) by experts.

The planned pose may be obtained by determining the planned pose based on the identified at least one anatomical element 22 and one or more predefined spatial constraints between the implant and the identified at least one anatomical element 22. The one or more predefined spatial constraints may define that the implant must be arranged, at least in part, within a pedicle of a vertebra. The planned pose may be determined by detecting a smallest (e.g., most narrow) portion of the pedicle of the vertebra based on the image data, and defining the planned pose relative to the detected smallest portion. The planned pose may be determined by defining an axis of the implant as extending through the detected smallest portion. The planned pose may be determined by detecting the superior endplate of the vertebra based on the image data, and defining the planned pose relative to the detected superior endplate. The planned pose may be determined by defining the axis of the implant as extending parallel to the detected superior endplate. The planned pose may be determined by defining the transverse angle of the axis of the implant according to the transverse angle of the pedicle. The planned pose may be determined based on the image data using an artificial neural network trained with image data sets of corresponding anatomical elements enriched with planned poses defined by experts.

In step 208, based on the image data, the implant data and the planning data, at least one first part of the implant that lies inside the at least one anatomical element 22 (e.g., as represented by the patient image data) and at least one second part of the implant does not lie inside the at least one anatomical element 22 (e.g., as represented by the patient image data) are determined.

In step 210, display of a visualization is triggered. The visualization indicates the at least one first part distinguishably from the at least one second part.

The method may comprise a step (e.g., preceding step 210) of determining the visualization based on the determined at least one first part and the determined at least one second part. The visualization may indicate the at least one first part and the at least one second part such that a human can distinguish the at least one first part from the at least one second part. The visualization may indicate the at least one first part distinguishably from the at least one second part by showing the first part or an outline of the first part with a first optical property and showing the second part or an outline of the second part with a different, second optical property. The optical property may be an optical highlighting, a color, a shading, an optical pattern, a contrast, a line thickness, a line pattern, a blinking pattern, an opacity or the like. The visualization may include a (e.g., user-assignable or predefined) marking, numbering or naming of one or both of (i) the at least one first part and (ii) the at least one second part. The visualization may include an indication of the at least one anatomical element 22, for example an indication (e.g., a partially transparent view) of a model of the at least one anatomical element 22 generated based on the patient image data using image segmentation.

The at least one second part may lie inside at least one zone separate from the at least one anatomical element 22. The at least one zone may comprise a zone distant from one or more of the at least one anatomical element 22. Alternatively, or in addition, the at least one zone may comprise a zone adjacent to one or more of the at least one anatomical element 22. As mentioned above, the method may comprise identifying the at least one anatomical element 22 in the image data. The method may comprise defining the at least one zone based on the identified at least one anatomical element 22. The at least one zone may border a side of the at least one anatomical element 22. The at least one zone may surround, encase or encircle the at least one anatomical element 22. The at least one zone may comprise or correspond to (e.g., a part of the patient's body comprising) the portion of the patient's body except for the at least one anatomical element 22. The at least one zone may comprise another implant such as a vertebral cage, a spinal rod or a pedicle screw. The visualization may include an indication of the at least one zone.

The visualization may indicate a first segment of the at least one second part that lies inside a first zone of the at least one zone distinguishably from a second segment of the at least one second part that lies inside a second zone of the at least one zone. In view of the distinguishability of the first segment and the second segment, the same as described above for the first part and the second part applies (e.g., using different colors). The first zone may comprise another anatomical element 24 (i.e., different from the at least one anatomical element 22). The second zone may comprise yet another anatomical element 26. The first zone may differ from the second zone, for example in at least one parameter chosen from size, shape, position and orientation. The first zone may differ from the second zone in that it comprises critical body structures such as artilleries, veins or nerves, whereas the second zone does not. The first zone may border a first side of the at least one anatomical element 22 and the second zone may border a second side of the at least one anatomical element 22, wherein the first side differs from, is adjacent to or is opposite to the second side. The first zone may correspond to a region confined by the at least one anatomical element 22, for example at two ends of the region. The first zone may correspond to a region surrounded or encased by at least a part of the at least one anatomical element 22. The second zone may correspond to a region exterior the at least a part of the at least one anatomical element 22. The at least one anatomical element 22 may be a vertebra and the first zone may correspond to (i) a region between the transverse process and the vertebral body, (ii) a region between the vertebral arch and the vertebral body, or (iii) the vertebral foramen. The at least one anatomical element 22 may be a vertebra and the second zone may correspond to (i) a region posterior the transverse process, (ii) a region lateral the spinous process, or (iii) a region anterior the vertebral body. The visualization may include an indication of the first zone. The visualization may include an indication of the second zone. The visualization may include a distinguishable indication of the first zone and the second zone. In view of the distinguishability of the first zone and the second zone, the same as described above for the first part and the second part applies (e.g., using different highlighting of the respective borders).

The visualization may indicate a third segment of the at least one second part that is comprised in a first predefined section of the implant distinguishably from a fourth segment of the at least one second part that is comprised in a second predefined section of the implant, wherein the first predefined section differs from the second predefined section. In view of the distinguishability of the third segment and the fourth segment, the same as described above for the first part and the second part applies (e.g., using different shading). The third segment may comprise an end (e.g., a longitudinal end, a distal end or a distal tip) of the implant. The fourth segment may comprise an opposite end (e.g., the other longitudinal end or the other distal end) of the implant. The implant may be configured to be inserted into the patient's body starting with the third segment. The fourth segment may comprise a tool attachment portion configured to couple to a surgical tool such as a screwdriver. The visualization may include an indication of the third segment. The visualization may include an indication of the fourth segment.

The visualization may be an augmented view. The visualization may visualize the at least one first part of the implant, the at least one second part of the implant and, optionally, the at least one anatomical element 22 in an overlaid manner with the portion of the patient's body 28 or a camera image of the portion of the patient's body 28. The zone(s) and segment(s) may also be visualized overlaid onto the portion of the patient's body 28 or a camera image of the portion of the patient's body 28. The camera image may be obtained by the sensor 16 of the ARD 300.

The method may comprise obtaining a first registration between (i) the image data and (ii) one or more of the at least one anatomical element 22. The method may further comprise obtaining a second registration between (i) the ARD 300 comprising the display 14 and (ii) one or more of the at least one anatomical element 22. The visualization may be determined based on the first registration and the second registration. The visualization may be triggered to be displayed on the display 14 of the ARD 300.

The first registration may be determined based on tracking data from the STS 400. Various techniques of determining the first registration based on the tracking data are possible. As an example, a plurality of points may be acquired on a surface of the patient's body 28 or on the at least one anatomical element 22, using a tracked registration probe, to obtain the tracking data. The plurality of points may then be mapped to the patient image data. This may yield the first registration, which may anchor the image data to the patient tracker. To account for movements of the at least one anatomical element after having obtained the first registration, the patient tracker 18 fixedly attached relative to the patient's body 28 may be (e.g., continuously) tracked, resulting in a corresponding shift of the anchored patient image data. As another example, the patient tracker 18 may comprise one or more fiducial markers and the image data may be generated by scanning the patient's body 28 and the patient tracker 18 simultaneously. The fiducial markers may then be identified in the image data to obtain the first registration, which may then be anchored to the patient tracker 18. Other variants of determining the first registration are also possible.

The second registration may be obtained by tracking the ARD 300 and the one or more of the at least one anatomical element 22 using the STS 400. As a first example, the ARD 300 may be provided with the tracker 20 and the STS 400 may track both the patient tracker 18 and the tracker 20 of the ARD 300. As a second example, the pose of the ARD 300 within an operating room may be determined using data acquired from the internal sensor(s) 16. The STS 400 may then be used to obtain a pose of the at least one anatomical element 22 within the operation room. The second registration may be determined based on the pose of the ARD 300 and the pose of the at least one anatomical element 22 within the operation room. As a still further example, a pose of the patient tracker 18 or the at least one anatomical element 22 relative to the ARD 300 may be determined based on sensor data acquired with internal sensor(s) 16 such as a camera, a depth sensor or the like. Other variants of determining the second registration are also possible.

The method may comprise obtaining a surface scan of the patient's body 28. The surface scan may consist of non-medical image data and may be separate from the patient image data. The surface scan may represent only the surface of at least the portion of the patient's body 28. The surface scan may be acquired by the sensor 16 comprised in the ARD 300. Alternatively, the surface scan may be acquired by a sensor separate from (e.g., external to) the ARD 300. The surface scan may be obtained from the STS 400. The surface scan may be obtained from a time-of-flight camera, a stereo camera, a video camera or the like.

The method may comprise a step of registering the surface scan to the image data or vice versa, for example based on a third registration (e.g., between (i) a coordinate system of the image data and (ii) a coordinate system of the surface scan or the sensor acquiring the surface scan). The third registration may be determined based on a tracked pose of the at least one anatomical element 22 (e.g., tracked via the tracker 20), the first registration between the image data and the at least one anatomical element 22, and a tracked pose of the sensor or scanner that acquires the surface scan. Other variants of determining the third registration are also possible.

The method may comprise determining, based on the surface scan and the planning data (e.g., and further based on the third registration), an incision point on the surface of the patient's body 28 at which an incision is to be made for inserting the implant into the patient's body 28. The visualization may further indicate the incision point.

The incision point may be indicated as a virtual marking on the surface of the patient's body 28. The incision point may be indicated by a symbol such as a point, a cross, an arrow or the like, which is for example overlaid onto the surface of the (e.g., portion of the) patient's body 28 or an image thereof. The indication of the incision point may include a numerical or textual description or marking of the incision point.

The incision point may be determined as an intersection between (i) a surface of the patient's body indicated by the surface scan and (ii) a trajectory having a predefined pose relative to the implant. The trajectory may be parallel or identical to a longitudinal axis of the implant. The trajectory may be parallel or identical to an axis around which the implant needs to be turned when being inserted into the at least one anatomical element 22. The visualization may include an indication of the trajectory.

The method may comprise determining whether the trajectory intersects one or more anatomical elements (e.g., including the at least one anatomical element 22). If the trajectory intersects the one or more anatomical elements, the visualization may include an indication of the trajectory intersecting the one or more anatomical elements (e.g., a highlighting of the intersected anatomical element(s)).

The method may comprise determining, based on the planning data (and, optionally, based on the implant data), a pose of a surgical instrument configured to couple to the implant to insert the implant into the patient's body. The surgical instrument may be a screwdriver. The method may comprise determining, based on the pose of the surgical instrument and based on one or more geometric properties of the surgical instrument (e.g., a size, shape or type thereof), whether the surgical instrument in the determined pose intersects one or more anatomical elements (e.g., including the at least one anatomical element 22). If the surgical instrument intersects the one or more anatomical elements, the visualization may include an indication of the surgical instrument intersecting the one or more anatomical elements, for example in the form of a warning message or by highlighting intersection areas or volumes between the surgical instrument and the one or more anatomical elements.
The method may further comprise, after having triggered display of the visualization, obtaining a user input indicative of an updated planned pose of the implant relative to the at least one anatomical element 22 (e.g., as represented by the image data). The method may comprise updating the visualization based on the updated planned pose. The user input may be obtained via the ARD 300. The user input may comprise a command gesture. The user input may be obtained via the sensor 16 comprised in the ARD 300, which is for example also used to acquire the surface scan. The user input may define a change of the planned pose of the implant, for example a change of the planned position of the implant, a change of the planned orientation of the implant or a change of the planned position and the planned orientation of the implant.

The visualization may guide a surgeon on how to adapt the planned pose of the implant. The visualization may guide a surgeon on how to adapt the planned pose by distinguishably visualizing the at least one first part from the at least one second part. The visualization may provide the surgeon with information on the first part of the implant that lies within the at least one anatomical element 22, and the second part of the implant that lies outside the at least one anatomical element 22 such that the surgeon may not need to cognitively determine these parts by himself. The visualization may guide the surgeon on how to adapt the pose of the implant such that the at least one second part is minimized in size, such that a portion of the at least one second part is changed into a portion of the at least one first part or such that the at least one first part is maximized in size, for example.

The sequence of the steps 202, 204 and 206 may be changed, as long as these steps are performed before step 208. Two or more of the steps 202, 204 and 206 may be combined in a single step. For example, two or more of the image data, the implant data and the planning data may be included in a single dataset obtained in a single step.

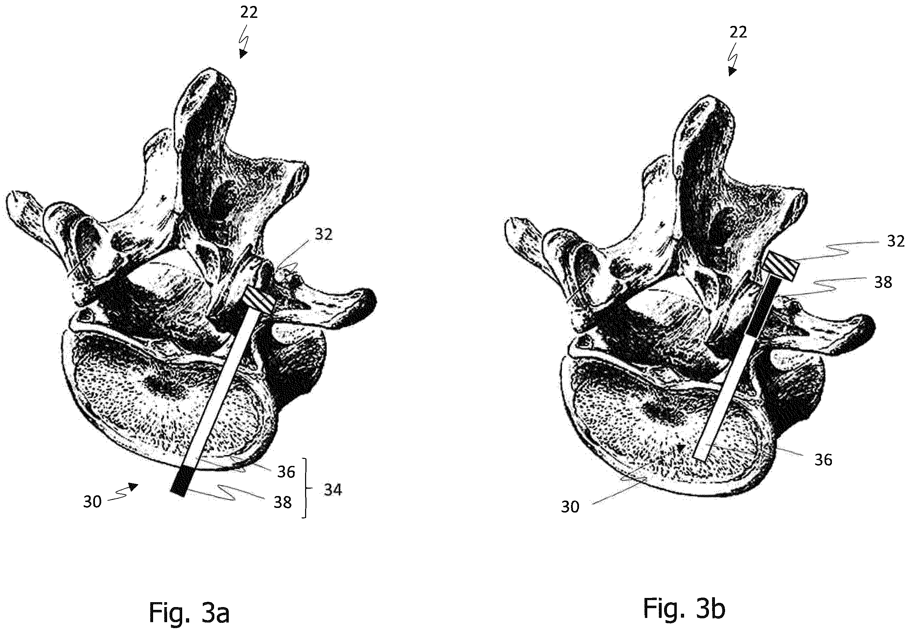

Fig. 3a-3f show visualizations in accordance with the present disclosure. In these examples, the implant 30 is a pedicle screw and the at least one anatomical element 22 is a vertebra. Each of the visualizations of Figs. 3a-3f may correspond to the visualization triggered to be displayed in step 210.

In the example of Fig. 3a, the screw 30 comprises a screw head 32, which may correspond to the third segment described herein. The screw 30 further comprises a shaft 34, which may correspond to the fourth segment described herein. The visualization indicates these two segments distinguishably. The planned pose defines the alignment between the implant 30 and the vertebra 22 as represented by the patient image data. The first part 36 of the implant 30, which lies inside the vertebra 22, is visualized distinguishably from the second part 38 of the implant 30, which lies outside the vertebra 22. In this case, the surgeon will note that the second part 38 of the implant 30 extends in the anterior direction beyond an outer surface of the vertebral body of the vertebra 22. Such a screw placement is usually not intended. The surgeon may thus adjust the planned pose such that the distal tip of the screw 30 no longer lies outside the vertebra 22, i.e., such that the highlighting of the screw's tip as second part 38 disappears.

In the example of Fig. 3a, the surgeon might move the implant 30 into the posterior direction. Upon receipt of a corresponding user command, the planned pose is updated. The resulting updated visualization is shown in Fig. 3b. As can be seen, the distal tip of the screw 30 is no longer highlighted as second part 38. However, a portion adjacent the screw head 32 is now highlighted as second part 38, which lies exterior to the vertebra 22. This is because the surgeon moved the screw 30 too far into the posterior direction, or because the length of the planned screw is too long. Note that also in the case of Fig. 3b, the screw head 32 may be visualized in a different manner than the second part 38 of the shaft 34, as the screw head 32 should always be arranged outside the vertebra 22.

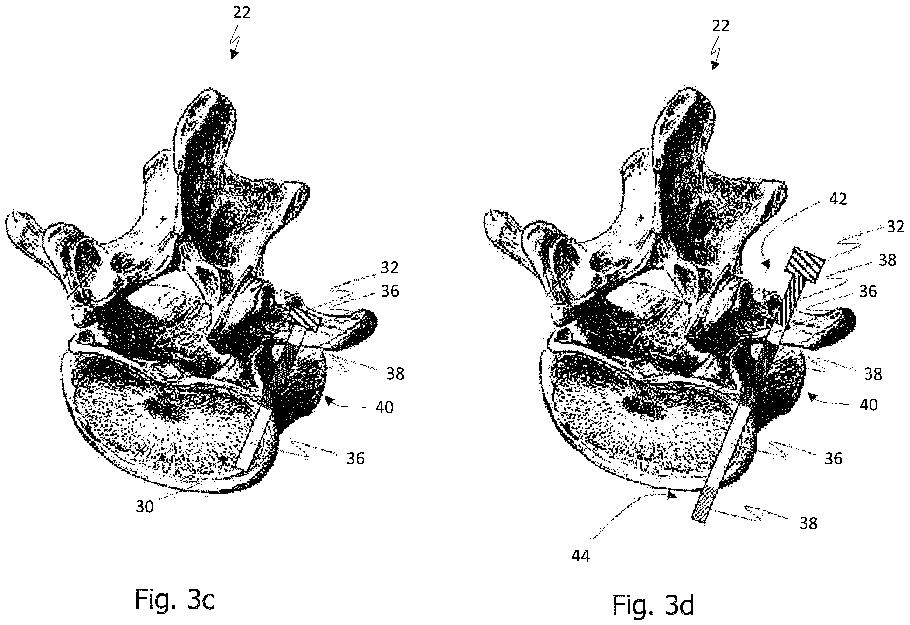

In some cases, the planned pose may result in a visualization as shown in Fig. 3c. In this scenario, there are multiple separate and distant first parts 36 of the implant 30, which each lie within the vertebra 22. All of these parts 36 may be indicated in the similar manner, for example using the same highlighting colour. The second part 38 in this case lies in a special zone 40, namely, in a region between the transverse process and the vertebral body. For that reason, the second part 38 may be highlighted in a different manner compared to the second part 38 shown in Fig. 3a and Fig. 3b.

Referring to Fig. 3d, the length of the screw 30 is larger compared with Fig. 3c. The distal tip of the screw 30 protrudes beyond the anterior surface of the vertebra 22. Thus, the distal tip represents a second part 38 of the screw 30, which lies outside the vertebra 22. At the other longitudinal end of the scree 30, another second part 38 of the implant 30 is highlighted. The second part 38 in the middle of the screw 30, which lies in the special zone 40, is highlighted similarly as in Fig. 3c. As can be seen, the second parts 38 are highlighted differently depending on whether or not they lie in the special zone 40. Multiple zones may be defined relative to the vertebra 22, for example the special zone 40, a posterior zone 42 and an anterior zone 44. The second part 38 may be highlighted differently depending on the zone in which it lies. The highlighting of the second parts 38 may also depend on their respective positions along the screw length. For instance, second parts 38 at the distal screw tip might be highlighted distinguishably from second parts 38 adjacent to the screw head 32, as second parts 38 at the distal screw tip generally pose a greater surgical risk.

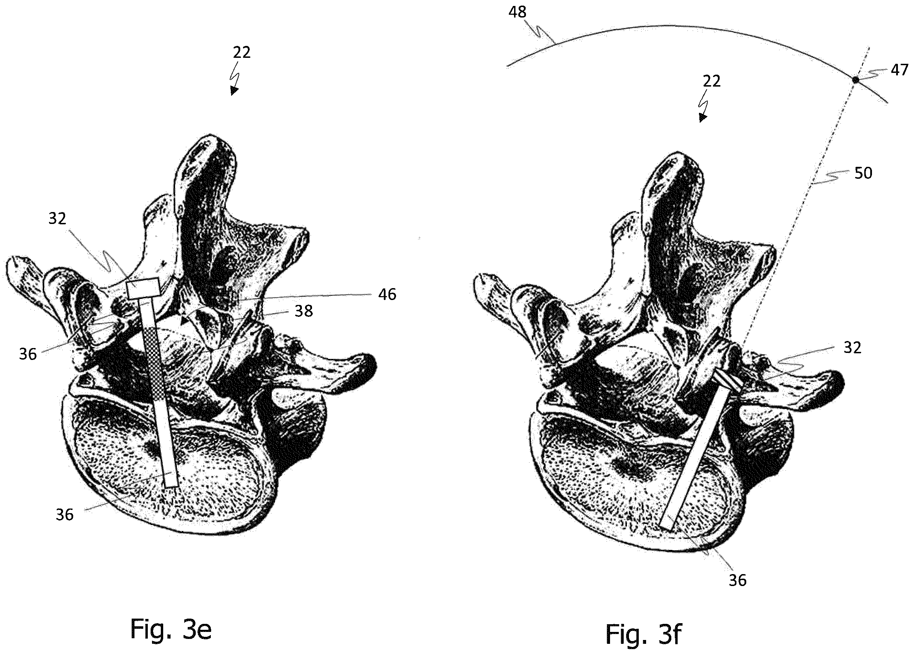

Fig. 3e shows another example in which the screw 30 partly extends within the vertebra 22 and partly outside thereof. In this example, the screw head 32 is highlighted in a similar manner as the first part 36 to focus the surgeon on the second part 38 of the screw 30. The second part 38 extends through the vertebral foramen. In this critical zone 46, nerve tissue of the spinal cord is arranged. Thus, a screw placement as shown in Fig. 3e should generally be avoided. The second part 38 may be highlighted in highly visible colours using a flashing highlighting. The surgeon may also be provided with a textual warning.

Due to the distinguishable highlighting of the first and second parts 36, 38 of the implant 30, the surgeon can easily recognize which parts of the implant 30 are aligned properly relative to the vertebra 22, and which parts might cause issues during or after surgery. He may then adjust the planned pose of the implant 30 accordingly using the visualization. The visualization may be continually updated upon adjusting the planned pose based on user feedback of the surgeon. This feedback loop may result in an optimal screw placement, as exemplarily illustrated in Fig. 3f.

The (e.g., adjusted or updated) planned pose of the implant 30 may be used to determine an incision point 47 on a surface 48 of the patient's body 28. In particular, the surface scan may be acquired and registered to the patient image data. This results in a known spatial relationship between the surface 48 of the patient's body 28 and the vertebra 22 as represented by the patient image data. The planned pose, which is for example defined relative to the patient image data, can then be used to determine, as the incision point 47, an intersection between (i) a trajectory 50 coinciding with the longitudinal screw axis of the pedicle screw 30 and (ii) the surface 48 as represented by the surface scan. The visualization triggered to be displayed may then comprise an indication of the incision point 47 and, optionally, an indication of the trajectory 50. The incision point 47 may lie on a region of the surface to that needs to be protected (i.e., that is not suitable for making an incision). The trajectory may extend through critical body tissue such as nerves, artilleries or veins. The surgeon may then adjust the planned pose once more to avoid a disadvantageous incision point or trajectory.

If the trajectory 50 intersects the vertebra 22 or an adjacent vertebra, the visualization may include an indication thereof. For example, all bones except for the vertebra 22, which are intersected by the trajectory, may be highlighted. Based on the adjusted pose of the implant 30, a pose of a surgical implant placement instrument may be determined. The pose of the surgical implant placement instrument may be an estimate in which pose the surgical implant placement instrument needs to be to position the implant 30 in the adjusted planned pose. The geometry of the surgical implant placement instrument may be known. This allows determining whether the surgical implant placement instrument, when placing the implant 30 at the adjusted planned pose, intersects one or more anatomical elements (e.g., including the vertebra 22). The visualization may include a visual highlighting of volumes of bones intersected by the surgical implant placement instrument. The surgeon may then decide whether to adapt the planned pose further, for example to avoid having to perform a bone resection before being able to place the implant 30 in its adjusted pose.

The surgeon may not only adjust the planned pose of the implant 30, but may alternatively or additionally adjust another property of the implant 30 (e.g., a type of the implant 30, a length of the implant 30 such as a screw length, a diameter of the implant 30 such as a screw diameter, or the like). The visualization may then be updated accordingly. Also in this case, the visualization may guide the surgeon on how to adapt the other property.

The incision point 47 may alternatively be determined in a distinct second method, which does not necessarily include the method steps described above with reference to Fig. 2. The second method may be performed by one or both of the processors 2, 8.

The second method comprises obtaining (e.g., the) planning data indicative of a (e.g., the) planned pose of an implant (e.g., the implant 30) relative to at least one anatomical element (e.g., 22) comprised in a portion of a patient's body (e.g., 28). The second method further comprises obtaining a (e.g., the) surface scan of at least the portion of the patient's body (e.g., 28). The second method comprises determining, based on the surface scan and the planning data, an incision point (e.g., 47) on the surface of the patient's body (e.g., 28) at which an incision is to be made for inserting the implant (e.g., 30) into the patient's body (e.g., 28). The method may comprise triggering display of a visualization indicating the incision point (e.g., 47). This visualization may be an augmented view and may be triggered to be displayed on the display 14 of the ARD 300. The incision point (e.g., 47) may be determined as an intersection between (i) a surface of the patient's body (e.g., 28) indicated or represented by the surface scan and (ii) a trajectory (e.g., 50) having a predefined pose relative to the implant (e.g., 30). The trajectory (e.g., 50) may coincide with a longitudinal axis of the implant (e.g., 30). The visualization may correspond to that illustrated in Fig. 3f.

The present disclosure may provide a visualization that informs a surgeon about which parts of a planned implant lie inside an anatomical element and which parts do not. The visualization may indicate whether the implant in the planned pose extends through critical zones comprising artilleries, nerve tissue and the like, or whether the implant is only arranged inside the first anatomical element as intended. The visualization may also inform the surgeon about an incision point that complies with the planned pose of the implant. An augmented view may improve the surgeon's understanding and perception of the relative spatial alignment between the planed implant and the anatomical element. By identifying the anatomical element in the patient image data, a special or critical zone may be determined without requiring the surgeon to designate the zones manually. The surgeon may plan the implant's planned pose in an image of the patient image data, obviating the need for a registration between the planned pose and the image data and thus saving processing effort. The pose may be determined automatically based on the image data, which means the surgeon does not need to plan the pose manually. The described technique may help the surgeon in judging whether the planned pose is acceptable or not. In particular, the visualization may guide a surgeon whether and how to adjust the planned pose, and updating the visualization may provide a feedback loop. Further advantages will be apparent to those skilled in the art in view of the above.

Modifications of the method(s) described herein are possible. For example, the second method may comprise all steps 202-210 described with reference to Fig. 2. As another example, planned pose may be defined in a planning coordinate system and may be transformed into the image coordinate system using a predefined transformation. The planned pose may be set by the surgeon using a tracked surgical instrument. In this case, the planned pose may be defined in the tracking coordinate system. The first registration may then be used to transform the planned pose into the image coordinate system. Other modifications of the present disclosure, for example using other coordinate systems and registrations, are also possible.

## Claims

1. A method for visualizing a planned implant (30), the method performed by a processing system (200) and comprising:
obtaining (202) image data representative of a portion of a patient's body (28), the portion comprising at least one anatomical element (22);
obtaining (204) implant data indicative of one or more geometrical properties of an implant (30) to be implanted into the at least one anatomical element (22);
obtaining (206) planning data indicative of a planned pose of the implant (30) relative to the at least one anatomical element (30);
determining (208), based on the image data, the implant data and the planning data, at least one first part (36) of the implant that lies inside the at least one anatomical element (22) and at least one second part (38) of the implant (30) does not lie inside the at least one anatomical element (22); and
triggering (210) display of a visualization indicating the at least one first part (36) distinguishably from the at least one second part (38).

2. The method of claim 1, wherein
the at least one second part (38) lies inside at least one zone separate 40; 42; 44) from the at least one anatomical element (22).

3. The method of claim 2, wherein at least one of the following conditions is fulfilled:
(i) the at least one zone (40; 42; 44) comprises a zone distant from one or more of the at least one anatomical element (22);
(ii) the at least one zone (40; 42; 44) comprises a zone adjacent to one or more of the at least one anatomical element (22).

4. The method of claim 2 or 3, wherein the visualization indicates a first segment of the at least one second part (38) that lies inside a first zone (40) of the at least one zone distinguishably from a second segment of the at least one second part (38) that lies inside a second zone (42; 44) of the at least one zone.

5. The method of any one of claims 1 to 4, wherein the visualization indicates a third segment of the at least one second part (38) that is comprised in a first predefined section of the implant (30) distinguishably from a fourth segment of the at least one second part (38) that is comprised in a second predefined section of the implant (30), wherein the first predefined section differs from the second predefined section.

6. The method of any one of claims 1 to 5, wherein
the visualization is an augmented view.

7. The method of claim 6, further comprising:
obtaining a first registration between (i) the image data and (ii) one or more of the at least one anatomical element (22); and
obtaining a second registration between (i) an augmented reality device (300) comprising a display (14) and (ii) one or more of the at least one anatomical element (22),
wherein the visualization is determined based on the first registration and the second registration,
wherein the visualization is triggered to be displayed on the display (14) of the augmented reality device (300).

8. The method of any one of claims 1 to 7, further comprising:
obtaining a surface scan of the patient's body (28); and
determining, based on the surface scan and the planning data, an incision point (47) on the surface (48) of the patient's body (28) at which an incision is to be made for inserting the implant (30) into the patient's body (28), wherein
the visualization further indicates the incision point (47).

9. The method of claim 8, wherein
the incision point (47) is determined as an intersection between (i) a surface (48) of the patient's body indicated by the surface scan and (ii) a trajectory (50) having a predefined pose relative to the implant (30).

10. The method of claim 8 or 9 when depending on claim 7, wherein
the surface scan is acquired by a sensor comprised in the augmented reality device (300).

11. The method of any one of claims 1 to 10, further comprising:
after having triggered display of the visualization, obtaining a user input indicative of an updated planned pose of the implant (30) relative to the at least one anatomical element (22); and
updating the visualization based on the updated planned pose.

12. The method of claim 2, or any one of claims 3 to 11 when depending on claim 2, further comprising:
identifying the at least one anatomical element (22) in the image data; and
defining the at least one zone (40; 42; 44) based on the identified at least one anatomical element (22).

13. The method of any one of claims 1 to 12, wherein
the planned pose is defined relative to the image data.

14. The method of claim 13, further comprising:
identifying the at least one anatomical element (22) in the image data; and
determining the planned pose based on the identified at least one anatomical element (22) and one or more predefined spatial constraints between the implant (30) and the identified at least one anatomical element (22).

15. The method of any one of claims 1 to 14, wherein at least one of the following conditions is fulfilled:
(i) the at least one anatomical element (22) comprises a bone such as a vertebra;
(ii) the implant (30) comprises a bone screw such as a pedicle screw.

16. A system (100) comprising at least one processor (2, 8) configured to perform a method for visualizing a planned implant, the at least one processor (2, 8) configured to:
obtain (202) image data representative of a portion of a patient's body (28), the portion comprising at least one anatomical element (22);
obtain (204) implant data indicative of one or more geometrical properties of an implant (30) to be implanted into the at least one anatomical element (22);
obtain (206) planning data indicative of a planned pose of the implant (30) relative to the at least one anatomical element (22);
determine, based on the image data, the implant data and the planning data, at least one first part (36) of the implant (30) that lies inside the at least one anatomical element (22) and at least one second part (38) of the implant (30) does not lie inside the at least one anatomical element (22); and
trigger display of a visualization indicating the at least one first part (36) distinguishably from the at least one second part (38).

17. The system (100) of claim 16, wherein the at least one processor (2, 8) is further configured to perform the method according to any one of claims 2 to 15.

18. The system (100) of claim 16 or 17, further comprising:
an augmented reality device (300) comprising a display (14),
wherein the at least one processor (2, 8) is configured to trigger display of the visualization on the display (14) of the augmented reality device (300).

19. The system (100) of claim 18, further comprising:
a tracking system (400) configured to track the patient's body (28) and the augmented reality device (300) to determine a relative pose between the patient's body (28) and the augmented reality device (300).

20. A computer program comprising instructions which, when executed on at least one processor (2, 8), cause the at least one processor (2, 8) to perform the method according to any one of claims 1 to 15.

## Amended claims

### Amended claims in accordance with Rule 137(2) EPC.

1. A method for visualizing a planned implant (30), the method performed by a processing system (200) and comprising:
obtaining (202) image data representative of a portion of a patient's body (28), the portion comprising at least one anatomical element (22);
obtaining (204) implant data indicative of one or more geometrical properties of an implant (30) to be implanted into the at least one anatomical element (22);
obtaining (206) planning data indicative of a planned pose of the implant (30) relative to the at least one anatomical element (30);
determining (208), based on the image data, the implant data and the planning data, at least one first part (36) of the implant that lies inside the at least one anatomical element (22) and at least one second part (38) of the implant (30) does not lie inside the at least one anatomical element (22); and
triggering (210) display of a visualization indicating the at least one first part (36) distinguishably from the at least one second part (38), the method further comprising:
identifying the at least one anatomical element (22) in the image data; and
determining the planned pose based on the identified at least one anatomical element (22) and one or more predefined spatial constraints between the implant (30) and the identified at least one anatomical element (22).

2. The method of claim 1, wherein
the at least one second part (38) lies inside at least one zone separate 40; 42; 44) from the at least one anatomical element (22).

3. The method of claim 2, wherein at least one of the following conditions is fulfilled:
(i) the at least one zone (40; 42; 44) comprises a zone distant from one or more of the at least one anatomical element (22);
(ii) the at least one zone (40; 42; 44) comprises a zone adjacent to one or more of the at least one anatomical element (22).

4. The method of claim 2 or 3, wherein the visualization indicates a first segment of the at least one second part (38) that lies inside a first zone (40) of the at least one zone distinguishably from a second segment of the at least one second part (38) that lies inside a second zone (42; 44) of the at least one zone.

5. The method of any one of claims 1 to 4, wherein the visualization indicates a third segment of the at least one second part (38) that is comprised in a first predefined section of the implant (30) distinguishably from a fourth segment of the at least one second part (38) that is comprised in a second predefined section of the implant (30), wherein the first predefined section differs from the second predefined section.

6. The method of any one of claims 1 to 5, wherein
the visualization is an augmented view.

7. The method of claim 6, further comprising:
obtaining a first registration between (i) the image data and (ii) one or more of the at least one anatomical element (22); and
obtaining a second registration between (i) an augmented reality device (300) comprising a display (14) and (ii) one or more of the at least one anatomical element (22),
wherein the visualization is determined based on the first registration and the second registration,
wherein the visualization is triggered to be displayed on the display (14) of the augmented reality device (300).

8. The method of any one of claims 1 to 7, further comprising:
obtaining a surface scan of the patient's body (28); and
determining, based on the surface scan and the planning data, an incision point (47) on the surface (48) of the patient's body (28) at which an incision is to be made for inserting the implant (30) into the patient's body (28), wherein
the visualization further indicates the incision point (47).

9. The method of claim 8, wherein
the incision point (47) is determined as an intersection between (i) a surface (48) of the patient's body indicated by the surface scan and (ii) a trajectory (50) having a predefined pose relative to the implant (30).

10. The method of claim 8 or 9 when depending on claim 7, wherein
the surface scan is acquired by a sensor comprised in the augmented reality device (300).

11. The method of any one of claims 1 to 10, further comprising:
after having triggered display of the visualization, obtaining a user input indicative of an updated planned pose of the implant (30) relative to the at least one anatomical element (22); and
updating the visualization based on the updated planned pose.

12. The method of claim 2, or any one of claims 3 to 11 when depending on claim 2, further comprising:
identifying the at least one anatomical element (22) in the image data; and
defining the at least one zone (40; 42; 44) based on the identified at least one anatomical element (22).

13. The method of any one of claims 1 to 12, wherein
the planned pose is defined relative to the image data.

14. The method of any one of claims 1 to 13, wherein at least one of the following conditions is fulfilled:
(i) the at least one anatomical element (22) comprises a bone such as a vertebra;
(ii) the implant (30) comprises a bone screw such as a pedicle screw.

15. A system (100) comprising at least one processor (2, 8) configured to perform a method for visualizing a planned implant, the at least one processor (2, 8) configured to:
obtain (202) image data representative of a portion of a patient's body (28), the portion comprising at least one anatomical element (22);
obtain (204) implant data indicative of one or more geometrical properties of an implant (30) to be implanted into the at least one anatomical element (22);
obtain (206) planning data indicative of a planned pose of the implant (30) relative to the at least one anatomical element (22);
determine, based on the image data, the implant data and the planning data, at least one first part (36) of the implant (30) that lies inside the at least one anatomical element (22) and at least one second part (38) of the implant (30) does not lie inside the at least one anatomical element (22); and
trigger display of a visualization indicating the at least one first part (36) distinguishably from the at least one second part (38), the at least one processor (2, 8) being further configured to:
identify the at least one anatomical element (22) in the image data; and
determine the planned pose based on the identified at least one anatomical element (22) and one or more predefined spatial constraints between the implant (30) and the identified at least one anatomical element (22).

16. The system (100) of claim 15, wherein the at least one processor (2, 8) is further configured to perform the method according to any one of claims 2 to 14.

17. The system (100) of claim 15 or 16, further comprising:
an augmented reality device (300) comprising a display (14),
wherein the at least one processor (2, 8) is configured to trigger display of the visualization on the display (14) of the augmented reality device (300).

18. The system (100) of claim 17, further comprising:
a tracking system (400) configured to track the patient's body (28) and the augmented reality device (300) to determine a relative pose between the patient's body (28) and the augmented reality device (300).

19. A computer program comprising instructions which, when executed on at least one processor (2, 8), cause the at least one processor (2, 8) to perform the method according to any one of claims 1 to 14.
